# EUROPÄISCHE PATENTANMELDUNG

(11) **EP 0 945 129 A2**
(43) Veröffentlichungstag der Anmeldung: **29.09.1999**
(21) Anmeldenummer: 99103841.5
(22) Anmeldetag: 27.02.1999
(51) Int. Cl.: A61K 7/48, A61K 31/25, A61K 31/765

(54) **Kosmetische und/oder pharmazeutische Zubereitungen**

(30) Priorität: 09.03.1998 DE 19810012
(71) Anmelder: Henkel Kommanditgesellschaft auf Aktien, 40589 Düsseldorf-Holthausen (DE)
(72) Erfinder: Ansmann, Achim, Dr. Dipl.-Chem., 40699 Erkrath (DE); Kawa, Rolf, 40789 Monheim (DE)

(57) **Zusammenfassung**

Vorgeschlagen werden kosmetische und/oder pharmazeutische Zubereitungen, enthaltend
(a) 0,1 bis 10 Gew.-% Polyolpoly-12-hydroxystearate,
(b) 0 bis 10 Gew.-% Alkyl- und/oder Alkenyloligoglykoside,
(c) 0,1 bis 20 Gew.-% Siliconverbindungen und
(d) 5 bis 20 Gew.-% niedere Alkohole oder Polyole.

Die Mittel zeichnen sich durch besonders vorteilhafte sensorische Eigenschaften aus.

## Beschreibung

### Gebiet der Erfindung

Die Erfindung betrifft gegen Verkeimung stabilisierte kosmetische und/oder pharmazeutische Zubereitungen mit einem Gehalt an ausgewählten Emulgatoren, Siliconverbindungen und niederen Alkanolen.

### Stand der Technik

Kosmetische bzw. pharmazeutische Emulsionen müssen üblicherweise durch Zusatz geeigneter Konservierungsmittel vor mikrobiellem Befall geschützt werden, um den Verbraucher vor gesundheitlichen Schäden, z.B. durch mikrobielle Toxine, zu bewahren. Konservierungsmittel wie beispielsweise Formalin oder Parabene können aber bei besonders sensibilisierten Personen Hautirritationen auslösen, so daß im Markt ein starkes Bedürfnis nach entsprechenden Zubereitungen besteht, die konservierungsmittelfrei und dennoch gegen Verkeimung stabil sind.

Eine Reihe von Herstellern sind daher dazu übergegangen, ihre Produkte frei von Konservierungsmitteln und unter besonders keimfreien Bedingungen herzustellen. In dem Augenblick, in dem der Verbraucher jedoch beispielsweise den Inhalt eines Tiegels mit den Fingern entnimmt, setzt die mikrobielle Kontaminierung des Produktes ein, die durch das Fehlen der Konservierungsstoffe nun ungebremst erfolgen kann. Demzufolge ist die Zeitspanne, binnen derer die Zubereitung verbraucht werden muß, äußerst gering. Alternativ dazu gibt es zwar entsprechende Dosierspender auf dem Markt, die einen direkten Kontakt zwischen der Zubereitung im Vorratsgefäß und der Haut des Verbrauchers vermeiden sollen, die Vorrichtungen sind jedoch sehr aufwendig und kostenintensiv. Eine weitere Möglichkeit besteht darin, mit Hilfe hochtouriger Homogenisatoren Wasser derart in Öl zu emulgieren, daß die dabei eingearbeiteten Wassertröpfchen etwa die Größe von Bakterien (ca. 1 mµ) haben. Ein Bakterium hat unter diesen Umständen nur dann eine Überlebenschance, wenn es in die unmittelbare Nähe eines Wassertröpfchens gerät und von dort Wasser, die darin gelösten Nährstoffe und Luft aufnehmen kann. Ist der Vorrat erschöpft, kommt es in der Regel zu einem raschen Absterben des Bakteriums, wobei es durch Wasserabgabe an die Umgebung austrocknet. Derartige Systeme sind zwar keimfrei, mikrobiologisch aber nicht belastbar, d.h. die Gefahr einer Kontamination bei Entnahme ist weiterhin gegeben. Gegen Verkeimung ausreichend stabilisierte W/O-Emulsionen können hingegen auch in Abwesenheit von Konservierungsmitteln hergestellt werden, indem man den Ölgehalt auf mindestens 70 Gew.-% anhebt. Derartige Produkte entsprechend hinsichtlich ihres Einziehvermögens sowie der Spreitung auf der Haut nicht den Anforderungen, die an moderne kosmetische Mittel gestellt werden. Für ein marktübliches Produkt kommt diese Möglichkeit daher nur sehr eingeschränkt in Frage. Schließlich ist es möglich, konservierungsmittelfreie, gegen Verkeimung stabilisierte W/O-Emulsionen herzustellen, indem man ihnen Polyole, vorzugsweise Glycerin in Mengen von mindestens 15 Gew.-% zusetzt. Diese Systeme sind jedoch in der Regel sensorisch ebenfalls sehr reichhaltig und belastend. Der alternative Einsatz von kurzkettigen Alkanolen, wie beispielsweise Ethanol, scheitert bislang daran, daß die Emulgatoren des Stands der Technik nicht in der Lage sind, entsprechende Mengen Alkohol über einen ausreichend langen Zeitraum zu emulgieren. Aus **Chem.Abstr. 115: 263077** und **Derwent-Abstr. 42 160C/24** sind Zubereitungen bekannt, die Ester von Polyolen mit monomerer 12-Hydroxystearinsäure enthalten. In **Chem.Abstr. 122: 268690** wird die Verwendung von Estern einer kondensierten 12-Hydroxystearinsäure mit Polyglycerin zur Solubilisierung von Tocopherol beschrieben. Aus **Chem.Abstr. 98: 8083** sind schließlich kosmetische Produkte auf Basis von 12-Hydroxystearinsäureoligoestern bekannt, die durch Veresterung von Poly-12-hydroxystearinsäure mit aliphatischen Alkoholen hergestellt werden.

Aus der deutschen Patentschrift **DE-C 19523478** (Henkel) sind konserveriungsmittelfreie Zubereitungen bekannt, die Emulgatoren vom Typ der Polyglycerinpoly-12-hydroxystearate zusammen mit Ethanol enthalten. Diese Mittel sind jedoch aus sensorischer Sicht nicht immer befriedigend.

Die komplexe Aufgabe der Erfindung hat somit darin bestanden, konservierungsmittelfreie kosmetische bzw. pharmazeutische Zubereitungen zur Verfügung zu stellen, die eine hohe Lagerstabilität auch bei höheren Temperaturen und eine ausreichend hohe Viskosität aufweisen gegenüber mikrobiellem Befall stabilisiert sind und insbesondere eine rasche Spreitung auf der Haut bewirken und einen angenehm leichten und samtigen Eindruck hinterlassen.

### Beschreibung der Erfindung

Gegenstand der Erfindung sind kosmetische und/oder pharmazeutische Zubereitungen, enthaltend
(a) 0,1 bis 10 Gew.-% Polyolpoly-12-hydroxystearate,
(b) 0 bis 10 Gew.-% Alkyl- und/oder Alkenyloligoglykoside,
(c) 0,1 bis 20 Gew.-% Siliconverbindungen und
(d) 5 bis 20 Gew.-% niedere Alkohole oder Polyole.

Überraschenderweise wurde gefunden, daß sich sowohl in W/O als auch O/W-Emulsionen, denen man als Emulgator Polyolpoly-12-hydroxystearate und insbesondere Polyglycerinpoly-12-hydroxystearat sowie gegebenenfalls Alkyl- und/oder Alkenyloligoglykoside zusetzt, auch größere Mengen Siliconöle und niedere Alkanole, insbesondere Ethanol in Mengen von jeweils bis zu 20 Gew.-% einarbeiten lassen, ohne daß es im Laufe der Lagerung zu einer Entmischung kommt. Die Produkte bleiben auch bei Lagerung bei Temperaturen im Bereich von 45°C über mindestens 3 Monate stabil, d.h. einphasig und sind in Abwesenheit von Konservierungsmitteln gegenüber mikrobiellem Befall ausreichend stabilisiert. Durch den Zusatz der Siliconverbindungen läßt sich zudem der sensorische Eindruck und die Spreitung der Produkte in unerwarteter Weise verbessern.

### Polyolpoly-12-hydroxystearate

Bei den Polyolpoly-12-hydroxystearaten handelt es sich um bekannte Stoffe, die beispielsweise unter der Marke Dehymuls® PGPH sowie in Abmischung mit Alkylglucosiden unter der Marke Eumulgin® VL 75 von der Henkel KGaA, Düsseldorf/FRG vertrieben werden. Die Stoffe werden beispielsweise in den deutschen Offenlegungsschriften **DE-A1 4420516** und **DE-A1 19533539** (Henkel) beschrieben. Die **Polyolkomponente** der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Wie schon erwähnt, kommen unter den erfindungsgemäß einzusetzenden Emulgatoren Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):
- Glycerin: : 5 bis 35 (15 bis 30) Gew.-%
- Diglycerine: : 15 bis 40 (20 bis 32) Gew.-%
- Triglycerine: : 10 bis 35 (15 bis 25) Gew.-%
- Tetraglycerine: : 5 bis 20 (8 bis 15) Gew.-%
- Pentaglycerine: : 2 bis 10 (3 bis 8) Gew.-%
- Oligoglycerine: : ad 100 Gew.-%

Die Einsatzmenge der Polyolpoly-12-hydroxystearate beträgt üblicherweise 0,1 bis 10 und vorzugsweise 1 bis 5 Gew.-% - bezogen auf die Zubereitungen.

### Alkyl- und/oder Alkenyloligoglykoside

Alkyl- und Alkenyloligoglykoside stellen bekannte nichtionische Tenside dar, die der Formel **(I)** folgen, 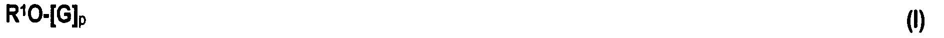 in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993),** B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Die Einsatzmenge der Alkyl- und/oder Alkenyloligoglykoside beträgt üblicherweise 0,1 bis 10 und vorzugsweise 1 bis 5 Gew.-% - bezogen auf die Zubereitungen. Die Komponenten (a) und (b) können dabei im Gewichtsverhältnis 90 : 10 bis 10 : 90, vorzugsweise 75 : 25 bis 25 : 75 und insbesondere 60 : 40 bis 40 : 60 eingesetzt werden.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Die Einsatzmenge der Siliconverbindungen beträgt üblicherweise 0,1 bis 20 und vorzugsweise 3 bis 10 Gew.-% - bezogen auf die Zubereitungen.

### Niedere Alkohole und Polyole

Als niedere Alkohole kommen aliphatische Alkohole mit 2 bis 4 Kohlenstoffatomen in Betracht. Typische Beispiele sind Propanol, Isopropylalkohol, tert.Butanol und vorzugsweise Ethanol. Des weiteren können auch Polyole, wie beispielsweise Ethylenglycol, 1,2-Propylenglycol, 1,4-Butylenglycol, 1,6-Hexylenglycol oder Glycerin eingesetzt werden. Die Einsatzmenge liegt im allgemeinen bei 5 bis 20 und insbesondere 10 bis 15 Gew.-% - bezogen auf die Zubereitungen.

### Kosmetische und/oder pharmazeutische Zubereitungen

Die erfindungsgemäßen Zubereitungen, wie beispielsweise Cremes, Lotionen oder Salben, können ferner als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Polymere, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen enthalten. Vorzugsweise sind die Mittel frei von Konservierungsmitteln.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremon- und -diester von Anlagerungsprodukten von 1 bis 30 Mol Ethylenoxid an Glycerin;
(3) Glycerinmono- und -diester und Sorbitanmon- und -diester von gesättigten und ungesättigten Fettsäuren mit 6 bis 22 Kohlenstoffatomen und deren Ethylenoxidanlagerungsprodukte;
(4) Anlagerungsprodukte von 15 bis 60 Mol Ethylenoxid an Ricinusöl und/oder gehärtetes Ricinusöl;
(5) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(6) Anlagerungsprodukte von 2 bis 15 Mol Ethylenoxid an Ricinusöl und/oder gehärtetes Ricinusöl;
(7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(8) Mono-, Di- und Trialkylphosphate sowie Mono-, Di- und/oder Tri-PEG-alkylphosphate und deren Salze;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmitte**l können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamin-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacryl/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zink-stearat bzw. -ricinoleat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppnmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtshutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäur-2-octylester und 4-(Dimethylamino)benzoesäureamylester;
- Ester der Zimtsäure, vorzugsweise 4-Methoxyzimtsäur-2-ethylhexylester, 4-Methoxyzimtsäureisopentylester, 2-Cyan-3-phenyl-zimtsäure-2-ethylhexylester (Octocrylene);
- Ester der Salicylsäure, vorzugsweise Salicylsäur-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Beispiele 1 bis 7, Vergleichsbeispiele V1 und V2.** Zur Herstellung verschiedener W/O- bzw. O/W-Lotionen bzw. Cremes wurden die Bestandteile I bei 80°C geschmolzen und homogen verrührt. Die Bestandteile der Phase II wurden ebenfalls auf 80°C erhitzt, langsam unter Rühren zu Phase I gegeben und 5 min bei 80°C emulgiert. Die Emulsion wurde unter Rühren abgekühlt und bei ca. 50°C vorsichtig homogenisiert. Nach Erreichen einer Temperatur von 30°C wurde das Rühren eingestellt und die Emulsionen nach Abkühlen auf Raumtemperatur entlüftet. Die Viskosität wurde unmittelbar nach der Herstellung sowie nach einer Lagerung von 4 Wochen bei 40°C in einem Brookfield RVT-Viskosimeter (10 Upm, Spindel 1) bestimmt. Die Spreitung auf der Haut sowie der sensorische Eindruck wurden durch ein Panel bestehend aus 6 geschulten Personen beurteilt. Dabei bedeuten: (++) sehr schnelle Spreitung, (++) schnelle Spreitung (+) übliche Spreitung und (o) sensorisch leichter, samtiger Eindruck bzw. (#) eher schwerer Eindruck. Die Ergebnisse sind in Tabelle 1 zusammengefaßt.

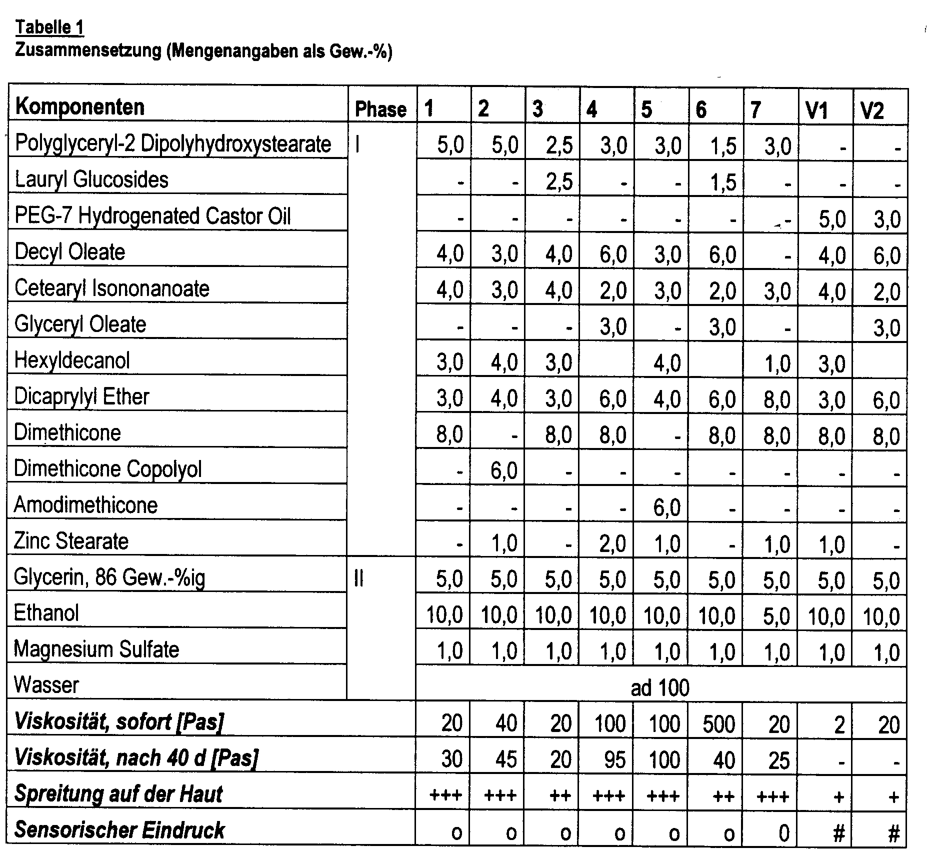

Bei den Formulierungen 1, 2, 4, 5, 7 sowie V1 und V2 handelt es sich um W/O-Emulsionen, die Formulierungen 3 und 6 stellen O/W-Emulsionen dar. Die Rezepturen 1 bis 3, 7 und V1 betreffen Lotionen, die Rezepturen 4 bis 6 und V2 Cremes. Ein direkter Vergleich der Formulierungen 1 und V1 bzw. 3 und V2 zeigt, daß bei Austausch des Emulgators Polyglyceryl-2 Dipolyhydroxystearate gegen PEG-7 Hydrogenated Castor Oil nicht nur die Viskosität um den Faktor 5 bis 10 abfällt, die Emulsionen sind auch bei Temperaturlagerung nicht mehr stabil, trennen sich, spreiten langsamer und hinterlassen einen eher schweren Eindruck auf der Haut.

## Patentansprüche

1. Kosmetische und/oder pharmazeutische Zubereitungen, enthaltend
(a) 0,1 bis 10 Gew.-% Polyolpoly-12-hydroxystearate,
(b) 0 bis 10 Gew.-% Alkyl- und/oder Alkenyloligoglykoside,
(c) 0,1 bis 20 Gew.-% Siliconverbindungen und
(d) 5 bis 20 Gew.-% niedere Alkohole oder Polyole.

2. Zubereitungen nach Anspruch 1, **dadurch gekennzeichnet**, daß sie als Komponente (a) Polyglycerinpoly-12-hydroxystearate enthalten.

3. Zubereitungen nach den Ansprüchen 1 und 2, **dadurch gekennzeichnet**, daß sie als Komponente (a) Polyglycerinpoly-12-hydroxystearate enthalten, deren Polyglycerinkomponente einen Eigenkondensationsgrad im Bereich von 2 bis 10 und die nachstehende Zusammensetzung aufweist:
Glycerin : 5 bis 30 Gew.-%
Diglycerine : 15 bis 40 Gew.-%
Triglycerine : 10 bis 30 Gew.-%
Tetraglycerine : 5 bis 20 Gew.-%
Pentaglycerine : 2 bis 10 Gew.-%
Oligoglycerine : ad 100 Gew.-%

4. Zubereitungen nach den Ansprüchen 1 bis 3, **dadurch gekennzeichnet**, daß sie als Komponente (b) Alkyl- und/oder Alkenyloligoglykoside der Formel **(I)** enthalten 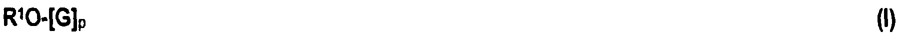 in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

5. Zubereitungen nach den Ansprüchen 1 bis 4, **dadurch gekennzeichnet**, daß sie die Komponenten (a) und (b) im Gewichtsverhältnis 90 : 10 bis 10 : 90 enthalten.

6. Zubereitungen nach den Ansprüchen 1 bis 5, **dadurch gekennzeichnet**, daß sie als Komponente (c) Siliconverbindungen enthalten, die ausgewählt sind aus der Gruppe, die gebildet wird von Dimethylpolysiloxanen, Methylphenylpolysiloxanen, cyclischen Siliconen sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können.

7. Zubereitungen nach den Ansprüchen 1 bis 6, dadurch gekennzeichnet, daß sie als Komponente (d) niedere Alkohole bzw. Polyole enthalten, die ausgewählt sind aus der Gruppe, die gebildet wird von Ethanol, Propanol, Isopropylalkohol, tert.Butanol, Ethylenglycol, 1,2-Propylenglycol, 1,4-Butylenglycol, 1,6-Hexylenglycol und Glycerin.

8. Zubereitungen nach den Ansprüchen 1 bis 7, **dadurch gekennzeichnet**, daß sie frei von Konservierungsmitteln sind.
